# EUROPEAN PATENT SPECIFICATION

(11) **EP 4 162 944 B1**
(45) Date of publication and mention of the grant of the patent: **04.11.2026**
(21) Application number: 21935430.5
(22) Date of filing: 15.09.2021
(51) Int. Cl.: A61K 36/54, A61P 31/04, A61P 1/02, A61P 1/10, A61K 8/9789, A61Q 11/00

(54) **PERIODONTAL DISEASE PREVENTIVE TREATMENT COMPOSITION**
ZUSAMMENSETZUNG ZUR VERBESSERUNG VON MUND UND ZAHNFLEISCH
COMPOSITION DE TRAITEMENT PRÉVENTIF DE MALADIE PARODONTALE

(30) Priority: 25.08.2021 JP 2021137231
(43) Date of publication of application: 12.04.2023
(73) Proprietor: Tokiwa Phytochemical Co., Ltd., Sakura-shi, Chiba 285-0801 (JP)
(72) Inventor: YANG, Jinwei, Sakura-shi, Chiba 285-0801 (JP); KUNIYOSHI, Tomoko, Sakura-shi, Chiba 285-0801 (JP); KOBAYASHI, Yukiko, Sakura-shi, Chiba 285-0801 (JP)
(74) Representative: Manitz Finsterwald Patent- und Rechtsanwaltspartnerschaft mbB
(86) International application number: PCT/JP2021/033851
(87) International publication number: WO 2023/026502

(56) References cited:
- JP-A- 2012 067 065
- JP-A- 2016 179 947
- JP-A- 2018 197 207
- CHIEKO INO ET AL: "Antimicrobial effects of Laurus nobilis leaf extract in combatting oral bacteria - Good oral health does contribute to good general health", JAPANESE JOURNAL OF CLINICAL ECOLOGY,, vol. 22, no. 1, 30 November 2012 (2012-11-30), pages 47 - 58, XP009543417
- TOMOKO KUNIYOSHI|ET AL. JAPANESE /: "The Effect of Ingesting of Foods Containing Laurus nobilis Leaf Extract(LAURESH )on Oral Environment in Healthy Subjects-A Randomized, Double-blind, Placebo-controlled Parallel-group Comparison Study-", 1 January 2021 (2021-01-01), XP093205399, Retrieved from the Internet <URL:https://www.pieronline.jp/content/article/0386-3603/49110/1913>
- MERGHNI A. ET AL: "Antibacterial and antibiofilm activities of Laurus nobilis L. essential oil against Staphylococcus aureus strains associated with oral infections", vol. 64, no. 1, 1 January 2016 (2016-01-01), pages 29 - 34, XP093205381, ISSN: 2452-3186, Retrieved from the Internet <URL:https://pdf.sciencedirectassets.com/313923/1-s2.0-S2452318616X00025/1-s2.0-S0369811415001017/main.pdf?X-Amz-Security-Token=IQoJb3JpZ2luX2VjENr//////////wEaCXVzLWVhc3QtMSJHMEUCIFEzKWm4nc4GsYs+/dqg+LC0c4WVajQdMOB5xWzpEdrYAiEApsaBn9+ipNtEMc6O3xbDuQMfY7eAGTJejpzqbBTwBHoqsgUIExAFGgwwNTkwMDM1NDY4NjUiDLoqq> DOI: 10.1016/j.patbio.2015.10.003
- FUKUYAMA NORIAKI ET AL: "Antimicrobial sesquiterpenoids from Laurus nobilis L.", NATURAL PRODUCT RESEARCH, vol. 25, no. 14, 1 August 2011 (2011-08-01), GB, pages 1295 - 1303, XP093205312, ISSN: 1478-6419, DOI: 10.1080/14786419.2010.502532
- PRIYADARSINI A ET AL: "Effectiveness of crushed bay leaves in removing dental plaque among tobacco chewer's", DRUG INVENTION TODAY (DIT), ELSEVIER BV, NETHERLANDS, vol. 14, no. 6, 1 June 2020 (2020-06-01), pages 1240 - 1244, XP009557079, ISSN: 0975-7619
- CHAUDHRY NAZIA MASOOD AHMED ET AL: "Bactericidal activity of black pepper, bay leaf, aniseed and coriander against oral isolates", PAKISTAN JOURNAL OF PHARMACEUTICAL SCIENCES,, vol. 19, no. 3, 1 July 2006 (2006-07-01), pages 214 - 218, XP009557080, ISSN: 1011-601X
- INO, CHIEKO ET AL.: "Antimicrobial effects of laurus nobilis leaf extract in combatting oral bacteria", JAPANESE JOURNAL OF CLINICAL ECOLOGY, vol. 22, no. 1, 30 November 2012 (2012-11-30), pages 47 - 58, XP009543389, ISSN: 0021-5007
- EVRENDILEK, G. AKDEMIR ET AL.: "Empirical prediction and validation of antibacterial inhibitory effects of various plant essential oils on common pathogenic bacteria", INT. J. FOOD MICROBIOL., vol. 202, 2015, pages 35 - 41, XP029123461, DOI: 10.1016/j.ijfoodmicro.2015.02.030
- ALEJO-ARMIJO, A ET AL.: "Antimicrobial and antibiofilm activities of procyanidins extracted from laurel wood against a selection of foodborne microorganisms", INT. J. FOOD SCI. TECHNOL, vol. 52, no. 3, 2017, pages 679 - 686, XP071857397, DOI: 10.1111/ijfs.13321

## Description

### [Field of the invention]

This invention is defined in the appended claims and relates to a composition for use in the treatment of periodontal disease, caused by periodontal disease-associated bacterium.

### [Background of the invention]

The bay tree (Laurus nobilis L.) belongs to the Lauraceae family, native to the Mediterranean, whose dried leaves, called laurier or laurel, have long been used as a spice in food. In the United States, the bay leaf is approved by the FDA (U.S. Food and Drug Administration) as GRAS (Generally Recognized As Safe), a safety standard given to food additives, and is approved as a food additive in Japan. In Europe, a decoction of bay leaves is used as a folk remedy for diarrhea, rheumatism, asthma, etc.

According to recent studies, extracts of bay leaves have been reported to have antibacterial effects against Porphyromonas gingivalis, one of the periodontal disease-associated bacteria (see Non-patent document 1), as well as neuroprotective, anti-allergic and anticonvulsant effects (see Non-patent document 2). Non-patent document 3 discloses that Laurus nobilis L. essential oils are able to inhibit oral Staphylococcus aureus strains. Non-patent document 4 describes compounds isolated from leaf extract of Laurus nobilis L. which show antimicrobial activity against periopathic pathogens. Non-patent document 5 mentions that crushed bay leaf powder possesses antimicrobial activity and helps to reduce dental plaque. Non-patent document 6 describes the antibacterial activity of aqueous decoction of Laurus nobilis L. against bacterial isolates from the oral cavity. Patent document 1 discloses an anti-periodontic disease medicine using a Laurus nobilis L. leaf extract as an effective component. Patent document 2 relates to an oral composition for prevention of periodontal disease which contains bay laurel extract.

### [Prior art documents]

### [Non-patent document]

[Non-patent document 1] Chieko Ino, Noriaki Fukuyama, Noritada Kobayashi, Yumiko Suzuki, Jinwei Yang and Yutaka Orihara, "Antimicrobial effects of Laurus nobilis leaf extract in combatting oral bacteria - Good oral health does contribute to good general health -," Japanese Journal of Clinical Ecology, Vol.22, No.1, pp.47-58 (2013).
[Non-patent document 2] Patrakar, R., Mansuriya, M., & Patil, P. (2012). Phytochemical and pharmacological review on Laurus nobilis. International journal of pharmaceutical and chemical sciences, 1(2), 595-602.
[Non-patent document 3] A. Merghni, H. Marzouki, H. Hentati, M. Aouni, M. Mastouri, Antibacterial and antibiofilm activities of Laurus nobilis L. essential oil against Staphylococcus aureus strains associated with oral infections, Pathologie Biologie, Vol. 64 (2016), pp. 29-34.
[Non-patent document 4] N. Fukuyama, C. Ino, Y. Suzuki, N. Kobayashi, H. Hamamoto, K. Sekimizu and Y. Orihara, Antimicrobial sesquiterpenoids from Laurus nobilis L., Natural Product Research, Vol. 25 (2011), No. 14, pp. 1295-1303.
[Non-patent document 5] A. Priyadarsini, R. Sugantha, Effectiveness of crushed bay leaves in removing dental plaque among tobacco chewers, Drug Invention Today, Vol. 14 (2020), Issue 7, pp. 1240-1244.
[Non-patent document 6] N. M. A. Chaudhry, P. Tariq, Bactericidal activity of black pepper, bay leaf, aniseed and coriander against oral isolates, Pak. J. Pharm. Sci., Vol. 19(3) (2006), pp. 214-218.

### [Patent documents]

[Patent document 1] JP 2012-067065 A
[Patent document 2] JP 2018-197207 A

### [Summary of the invention]

### [Problems to be solved by the Invention]

Although the bay tree has been reported to have the above effects, it is also expected to have new improving effects on the oral condition that has not previously been reported.

In view of the above issues, the present invention aims to provide a composition that can demonstrate new effects of the bay tree (Laurus nobilis L.).

### [Means to solve the problem]

The inventors have conducted research on the bay tree (Laurus nobilis L.) to solve the above issues, and as a result, the inventors have completed the present invention by demonstrating antibacterial effects against periodontal disease-associated bacterium Tannerella forsythensis (and, outside the scope of the claims, against dental caries-associated bacterium Streptococcus mutans), improving effects on gum, improving effects on gum ridge, inhibiting effects on plaque accumulation, improving effects on xerostomia, improving effects on saliva, inhibiting effects on bad breath, and improving effects on constipation of the bay tree (Laurus nobilis L.) in human tests.

According to the present invention, the composition for use in the treatment of periodontal disease, caused by periodontal disease-associated bacterium Tannerella forsythensis, contains bay tree (Laurus nobilis L.) extract as an active ingredient.

### [Effects of the invention]

As described above, the present invention provides a composition for use in the treatment of periodontal disease, caused by periodontal disease-associated bacterium Tannerella forsythensis.

### [Description of embodiments]

The embodiment will be explained in detail below. Note that the invention can be implemented in many different embodiments and is limited only by the appended claims.

This embodiment exhibits a composition for use in the treatment of periodontal disease, caused by periodontal disease-associated bacterium Tannerella forsythensis, that has antibacterial effects on Tannerella forsythensis (and, outside the scope of the claims, on Streptococcus mutans), improves the health of gum and gum ridge, xerostomia, and salivary pH, inhibits plaque accumulation and bad breath, improves constipation, and specifically, can be an antibacterial agent against periodontal disease-associated bacterium or dental caries-associated bacterium, a composition for periodontal disease prophylaxis and treatment, a composition for cavity prophylaxis and treatment, a composition for gum improvement, a composition for gum ridge improvement, a composition for inhibiting plaque accumulation, a composition for xerostomia improvement, a composition for saliva improvement, a composition for inhibiting bad breath, or a composition for constipation improvement, containing bay tree (Laurus nobilis L.) extract as an active ingredient.

In this embodiment, the part of the bay tree (Laurus nobilis L.) used as raw material is not limited to, but preferably the leaves, as long as the above effects can be obtained. The above part of the bay tree may be fresh or dried. The leaves may also be processed.

The method of extracting bay tree extract is not limited and can be carried out according to methods well known to those skilled in the art, specifically, preferably by solvent extraction. Cold or warm water, alcoholic solvents, acetone or other organic solvents can be used as extraction solvents. Examples of alcoholic solvents include methanol, ethanol, propanol, isopropanol, butanol, isobutanol and mixtures thereof. Examples of organic solvents other than acetone include esters such as ethyl acetate; polyhydric alcohols such as ethylene glycol, propylene glycol and 1,3-butylene glycol; and ethers such as diethyl ether. These solvents may be used alone or in combination. The extraction solvent may be a combination of water and a hydrophilic organic solvent in the form of a hydrophilic solvent containing water. When using a combination of solvents, the mixing ratio of solvents can be set arbitrarily. Among these, the use of ethanol is preferred, and the use of hydrous ethanol with an ethanol concentration of between 30% and 95% (v/v) is more preferred.

The amount of the extraction solvent is not limited as long as the composition exhibiting the above effects can be obtained, but for example, it is preferably between 2 wt. part and 100 wt. part per 1 wt. part of dried bay leaves. The extraction temperature is preferably between 4°C and 90°C. The extraction time is preferably between 30 minutes and one week. The extraction method can be any method such as agitation extraction, immersion extraction, countercurrent extraction, ultrasonic extraction and supercritical extraction.

The bay tree extract may be the filtrate itself obtained by filtering the extract, a concentrated solution obtained by concentrating the filtrate, a dried product obtained by drying the concentrated solution, a crudely purified or finely purified product of any of these, or a mixture of any of these. Concentration can be carried out by any method, such as evaporative concentration, membrane concentration, etc. Drying can be carried out by any method, such as decompression drying, freeze-drying, spray-drying, etc. Excipients such as dextrin may be added if necessary. Purification can be carried out according to means known to those skilled in the art. For example, synthetic adsorption resins, activated carbon, ion-exchange resins, gel filtration agents such as Sephadex and bio-gels, column chromatography, recrystallization, etc. may be used alone or in combination.

The compositions can take the form of a food composition, a pharmaceutical composition, a quasi-drug composition, or a cosmetic composition, and particularly in the case of a food composition, they can take the form of a food with function claims or a health food.

When the composition is a food composition, it can take the form of a solid as well as a fluid (for example, a liquid such as a beverage). For example, the form of the food composition can be a drink, a candy, a jelly, a gummi candy, a gum, etc. When it is a health food or a food with function claims, the form can be a tablet, a chewable tablet, a hard capsule, a soft capsule, a granule, a drink, etc.

When the composition is a pharmaceutical composition, it can also take the form of a solid as well as a fluid (for example, a liquid). For example, the form can be a jelly, a chewable, a tablet, a capsule, a pill, a liquid, an emulsion, etc. The method of administration is not limited, but it is preferably in a form that allows it to stay in the oral cavity for a longer period of time. Various carriers can also be added to the extent that they are pharmaceutically acceptable. For example, the carriers can be an excipient, a coloring agent, a sweetening agent, a suspending agent, etc.

When the composition is a quasi-drug composition, it can also take the form of a solid as well as a fluid (for example, a liquid) in the same manner as described above. For example, the form can be a toothpaste, a mouthwash, a mouth rinse, a paste, a gel, a spray, a sheet or any other form that can be applied in the oral cavity.

When the composition is a cosmetic composition, it can also take the form of a solid as well as a fluid in the same manner as described above. For example, the form can be a lotion, a cream, a pack, a gel, an emulsion, a balsam, an ointment, a liquid, a powder or a form that can be applied in the oral cavity such as a toothpaste, a mouthwash, a mouth rinse, a paste, a gel, a spray, a sheet, etc.

The content of bay tree (Laurus nobilis L.) extract in the composition can be adjusted according to the expected intake.

The amount of bay tree (Laurus nobilis L.) extract necessary to provide antibacterial effects against Tannerella forsythensis (and, outside the scope of the claims, against Streptococcus mutans), inhibiting effects on gum inflammation, spontaneous bleeding of gums and plaque accumulation, improving effects on xerostomia and salivary pH, inhibiting effects on bad breath, and improving effects on constipation with the composition is not limited as long as the effects of the composition can be provided, but it is preferably between 10 mg and 500 mg per day, more preferably between 20 mg and 300 mg per day, and even more preferably between 50 mg and 150 mg per day.

The bay tree extract in the compositions contains deacetyl laurenobiolide in the bay tree extract, and the content of deacetyl laurenobiolide is not limited to, but preferably 0.01 wt% or more, and more preferably between 0.1 wt% and 10 wt%. Inclusion in this range will preferably demonstrate the effects of the compositions.

As described above, the compositions have antibacterial effects against Tannerella forsythensis (and, outside the scope of the claims, against Streptococcus mutans), inhibiting effects on gum inflammation, spontaneous bleeding of gums and plaque accumulation, improving effects on xerostomia and salivary pH, inhibiting effects on bad breath, and improving effects on constipation. The range in which these effects can be obtained is also the same as above, as will be clear from the description of the example below.

As described above, the invention provides compositions for use in the treatment of periodontal disease, caused by periodontal disease-associated bacterium Tannerella forsythensis, that have antibacterial effects against Tannerella forsythensis (and, outside the scope of the claims, against Streptococcus mutans), inhibiting effects on gum inflammation, spontaneous bleeding of gums and plaque accumulation, improving effects on xerostomia and salivary pH, inhibiting effects on bad breath, and improving effects on constipation.

### [Example]

Here, the compositions according to the above embodiment were actually produced and their effects were tested. The following is a specific explanation.

### (1) Production of bay tree extract

100 g of bay tree (Laurus nobilis L.) leaves were mixed with 1.4 L of 85% (v/v) ethanol, heated to reflux for 2 hours and filtered. The extracted liquid was vacuum concentrated at 50°C and freeze-dried to yield the bay tree extract.

The liquid chromatographic measurement of the extract obtained above demonstrated that the bay tree extract contained 1.1 wt% of deacetyl larurenobiolide.

### (2) Clinical trials

Chewable tablets containing 18 mg of bay tree extract per tablet were used as the test food. Note that for placebo, dextrin was added instead of bay tree extract.

A randomized, placebo-controlled, double-blind, parallel-group trial was conducted in healthy Japanese adult male and female subjects. The subjects ingested three tablets of the test food per day, licking them without chewing them at any given point in time. The daily intake of bay tree extract in the intervention group was 108 mg. Each examination was conducted before and four weeks after the start of intake. Among them, for salivary pH, oral water content, saliva volume, halitosis and the questionnaire, the examination was conducted after a single intake of three tablets of the test food, to evaluate the effect of a single intake. The single intake test was conducted after the completion of the examination before the start of intake.

### (3) Measurement of oral Tannerella forsythensis

Saliva samples were collected from the subjects to evaluate the ratio of Tannerella forsythensis in the oral cavity. Comparison with the control group showed a significant improvement in the ratio of Tannerella forsythensis to total bacterial counts in the intervention group four weeks after the start of intake (Table 1). This result indicates that the bay tree extract has antibacterial effects against the periodontal disease-causing bacterium Tannerella forsythensis.

**[Table 1]**

| Item | Before intake | | P value | Four weeks after intake | | P value |
|---|---|---|---|---|---|---|
| | Intervention group | Control group | | Intervention group | Control group | |
| Ratio of Tannerella forsythensis to total bacterial counts | 0.010 ±0.009 | 0.008 ±0.010 | 0.388 | 0.008 ±0.008 | 0.014 ±0.023 | 0.028 |

### (4) Measurement of oral Streptococcus mutans (outside the scope of the claims)

Saliva samples were collected from the subjects to evaluate the number of Streptococcus mutans in the oral cavity. Comparison with the control group showed a significant improvement in the number of Streptococcus mutans in the intervention group four weeks after the start of intake (Table 2). This result indicates that the bay tree extract has antibacterial effects against the dental caries-causing bacterium Streptococcus mutans.

**[Table 2]**

| Item | Before intake | | P value | Four weeks after intake | | P value |
|---|---|---|---|---|---|---|
| | Intervention group | Control group | | Intervention group | Control group | |
| Number of Streptococcus mutans (CFU/swab) | 23665.7 ±55912.0 | 55650. 0 ±66077.5 | 0.413 | 4521. 4 ±5538.1 | 40800.0 ±23070.8 | 0. 006 |

### (5) Evaluation of gingival index (GI)

The gingival index (GI) was evaluated as an indicator of gum inflammation. The marginal gingiva of each tooth was divided into four areas (buccal, lingual, mesial and distal) to evaluate the inflammation on four levels: 0 (normal), 1 (mild redness and mild inflammation), 2 (redness, swelling and moderate inflammation) and 3 (strong inflammation and severe inflammation), determining GI with the formula "GI = sum of scores for each tooth area/number of teeth tested." Comparison with the control group showed a significant decrease in GI in the intervention group four weeks after the start of intake (Table 3).

**[Table 3]**

| Item | Before intake | | P value | Four weeks after intake | | P value |
|---|---|---|---|---|---|---|
| | Intervention group | Control group | | Intervention group | Control group | |
| GI | 1.8±1.5 | 2.1±1.0 | 0. 617 | 0.9±0.8 | 1.9±1.1 | 0.038 |

### (6) Evaluation of spontaneous bleeding of gums

Spontaneous bleeding of gums was evaluated as an indicator of the inflammatory condition of the gums. Each tooth was divided into four areas to check for bleeding of gums, and the number of bleeding spots, if observed, was counted to determine the total number of such spots. Comparison with the control group showed a significant decrease in the number of spontaneous bleeding spots on the gums in the intervention group four weeks after the start of intake (Table 4).

**[Table 4]**

| Item | Before intake | | P value | Four weeks after intake | | P value |
|---|---|---|---|---|---|---|
| | Intervention group | Control group | | Intervention group | Control group | |
| Number of spots (areas) of spontaneous bleeding of gums | 2. 8±7. 6 | 3. 4±5. 6 | 0.780 | 0. 9±2. 5 | 3. 3±5. 4 | 0.053 |

### (7) Evaluation of plaque index (PlI)

The plaque index (PlI) was evaluated as an indicator of plaque accumulation. Each tooth was divided into four areas to determine the extent of plaque buildup on four levels: 0 (no plaque), 1 (a thin film of plaque adherent to the free gingival margin and interdental space on the tooth surface, which can be identified by the use of a stain solution or a probe), 2 (moderate accumulation of soft deposits discernible to the naked eye) and 3 (large amounts of soft deposits in the gingival sulcus, on the tooth surface or on the gingival margin), determining PlI by summing the scores of the four areas and dividing by four. Comparison with the control group showed a significant decrease in PlI in the intervention group four weeks after the start of intake (Table 5).

**[Table 5]**

| Item | Before intake | | P value | Four weeks after intake | | P value |
|---|---|---|---|---|---|---|
| | Intervention group | Control group | | Intervention group | Control group | |
| PlI | 0.8±0.3 | 0.9±0.4 | 0. 306 | 0.4±0.2 | 0.8±0.3 | 0.005 |

### (8) Measurement of oral water content

An oral moisture meter was used to measure the subjects' oral water content. Comparison with the control group showed a significant increase in oral water content with a single intake in the intervention group (Table 6).

**[Table 6]**

| Item | Change (after single intake - before intake) | | P value |
|---|---|---|---|
| | Intervention group | Control group | |
| Oral water content | 1.6±3.6 | -0.8±2.3 | 0. 036 |

### (9) Measurement of saliva volume

Saliva was collected from the oral cavity in a resting state for 15 minutes to measure the weight of saliva as an indicator of saliva volume. While there was no significant difference in saliva volume before and after a single intake in the control group, the intervention group showed a significant increase in saliva volume after a single intake (Table 7).

**[Table 7]**

| Item | Intervention group | | P value | Control group | | P value |
|---|---|---|---|---|---|---|
| | Before intake | After single intake | | Before intake | After single intake | |
| Amount of saliva (g) | 5.1±2.7 | 6.5±2.7 | 0.0001 | 5.5±3.4 | 6.2±2.7 | 0. 164 |

### (10) Questionnaire on saliva volume

The Likert scale method was used to evaluate saliva volume by examining the item "small amount of saliva" in the questionnaire. The item in the questionnaire was answered on a six-point scale from 1 to 6 (1: Not at all applicable, 2: Mostly not applicable, 3: Not very applicable, 4: Slightly applicable, 5: Somewhat applicable and 6: Very applicable), determining a score. While there was no significant difference in the score for the item "small amount of saliva" before and after a single intake in the control group, the intervention group showed a significant improvement in the score after a single intake (Table 8).

**[Table 8]**

| Item | Group | Before intake | | | After single intake | | | P value |
|---|---|---|---|---|---|---|---|---|
| | | Median | Q1 | Q3 | Median | Q1 | Q3 | |
| Score for "small amount of saliva" | Intervention group | 3.0 | 2.0 | 3.0 | 2.0 | 2.0 | 3.0 | 0.045 |
| | Control group | 3.0 | 2.0 | 4.0 | 3.0 | 2.0 | 3.0 | 0. 254 |

| | | | | | | | | |
|---|---|---|---|---|---|---|---|---|
| Q1: first quartile, Q3: third quartile | | | | | | | | |

The results in Tables 6, 7 and 8 indicate that bay tree extract increases the oral water content and improves xerostomia.

### (11) Measurement of salivary pH

Salivary pH was measured on saliva collected from the subjects using litmus test paper. Comparison with the control group showed a significant improvement in salivary pH in the intervention group after a single intake (Table 9). This result indicates that bay tree extract has the effect of increasing salivary pH.

**[Table 9]**

| Item | Before intake | | P value | After single intake | | P value |
|---|---|---|---|---|---|---|
| | Intervention group | Control group | | Intervention group | Control group | |
| Salivary pH | 6.9±0.4 | 6. 9±0.5 | 0.923 | 7.1±0.5 | 6.9±0.5 | 0.030 |

### (12) Measurement of halitosis

The amount of volatile sulfur compounds in the oral cavity, the main cause of halitosis, was measured as an indicator of halitosis by using a halitosis measuring device. While there was no significant change in halitosis before and after a single intake in the control group, the intervention group showed a significant decrease in halitosis after a single intake (Table 10). This result indicates that bay tree extract has inhibiting effect on bad breath.

**[Table 10]**

| Item | Intervention group | | P value | Control group | | P value |
|---|---|---|---|---|---|---|
| | Before intake | After single intake | | Before intake | After single intake | |
| Halitosis (ppb) | 124. 7 ±38.7 | 82. 1 ±10.5 | 0.017 | 193.8 ±90. 2 | 125. 8 ±65. 9 | 0. 210 |

### (13) Questionnaire on constipation

The Likert scale method was used to evaluate constipation by examining the item " constipation" in the questionnaire. The item in the questionnaire was answered on a six-point scale from 1 to 6 (1: Not at all applicable, 2: Mostly not applicable, 3: Not very applicable, 4: Slightly applicable, 5: Somewhat applicable and 6: Very applicable), determining a score. Comparison with the control group showed a significant improvement in the score of the item " constipation" in the questionnaire in the intervention group four weeks after the start of intake (Table 11).

**[Table 11]**

| Item | Group | Before intake | | | Four weeks after intake | | | P value |
|---|---|---|---|---|---|---|---|---|
| | | Median | Q1 | Q3 | Median | Q1 | Q3 | |
| Score for "constipation" | Intervention group | 2.0 | 1.0 | 3.0 | 1.0 | 1.0 | 2.0 | 0.021 |
| | Control group | 2.0 | 2.0 | 3.3 | 1.5 | 1.0 | 2.5 | 0.086 |

The above human tests showed that the compositions have antibacterial effects against Tannerella forsythensis (and, outside the scope of the claims, against Streptococcus mutans), inhibiting effects on gum inflammation, spontaneous bleeding of gums and plaque accumulation, improving effects on xerostomia and salivary pH, inhibiting effects on bad breath, and improving effects on constipation.

## Claims

1. A composition for use in the treatment of periodontal disease, caused by periodontal disease-associated bacterium Tannerella forsythensis, containing bay tree (Laurus nobilis L.) extract as an active ingredient.

2. A food composition containing bay tree (Laurus nobilis L.) extract as an active ingredient for use according to claim 1.

3. A quasi-drug composition containing bay tree (Laurus nobilis L.) extract as an active ingredient for use according to claim 1.

4. A cosmetic composition containing bay tree (Laurus nobilis L.) extract as an active ingredient for use according to claim 1.

## Patentansprüche

1. Zusammensetzung zur Verwendung bei der Behandlung einer Parodontalerkrankung, verursacht durch das mit Parodontalerkrankung assoziierte Bakterium Tannerella forsythensis, die einen Extrakt aus Lorbeerbaum (Laurus nobilis L.) als Wirkstoff enthält.

2. Lebensmittelzusammensetzung, die einen Extrakt aus Lorbeerbaum (Laurus nobilis L.) als Wirkstoff enthält, zur Verwendung nach Anspruch 1.

3. Quasi-Arzneimittelzusammensetzung, die einen Extrakt aus Lorbeerbaum (Laurus nobilis L.) als Wirkstoff enthält, zur Verwendung nach Anspruch 1.

4. Kosmetische Zusammensetzung, die einen Extrakt aus Lorbeerbaum (Laurus nobilis L.) als Wirkstoff enthält, zur Verwendung nach Anspruch 1.

## Revendications

1. Composition pour utilisation dans le traitement d'une maladie parodontale causée par la bactérie Tannerella forsythensis associée aux maladies parodontales, contenant un extrait de laurier (Laurus nobilis L.) en tant que principe actif.

2. Composition alimentaire contenant un extrait de laurier (Laurus nobilis L.) en tant que principe actif pour utilisation selon la revendication 1.

3. Composition de quasi-médicament contenant un extrait de laurier (Laurus nobilis L.) en tant que principe actif pour utilisation selon la revendication 1.

4. Composition cosmétique contenant un extrait de laurier (Laurus nobilis L.) en tant que principe actif pour utilisation selon la revendication 1.
